# EUROPEAN PATENT SPECIFICATION

(11) **EP 1 499 278 B1**
(45) Date of publication and mention of the grant of the patent: **01.06.2005**
(21) Application number: 04737220.6
(22) Date of filing: 19.05.2004
(51) Int. Cl.: A61K 6/00, A61K 9/20, A61K 31/567

(54) **IMMEDIATE-RELEASE PHARMACEUTICAL DOSAGE FORM COMPRISING POLYMORPHOUS TIBOLONE**
SCHNELL FREISETZENDE FARMAZEUTISCHE DARREICHUNGSFORM ENTHALTEND POLYMORPHES TIBOLON
FORMULATION SOLIDE PHARMACEUTIQUE CONTENANT DE LA TIBOLONE

(30) Priority: 23.05.2003 EP 03101500
(43) Date of publication of application: 26.01.2005
(73) Proprietor: Akzo Nobel N.V., 6824 BM Arnhem (NL)
(72) Inventor: DE HAAN, Pieter, N.V. Organon, 5340 BH Oss (NL)
(74) Representative: Van den Broek, Ludovicus A.G.M.
(86) International application number: PCT/EP2004/050852
(87) International publication number: WO 2004/103378

(56) References cited:
- EP-A- 0 389 035
- WO-A-03/032924
- US-A- 4 196 188
- US-A1- 2001 005 513
- DECLERCQ J P ET AL: "Conformational analysis of 3-oxo 5(10)-unsaturated steroids. Single-crystal x-ray structure analysis of 17-hydroxy-7.alpha.-methyl -19-nor-17.alpha.-pregn-5(10)-en-20-yn-3-o ne (Org OD 14)" May 1984 (1984-05), RECUEIL DES TRAVAUX CHIMIQUES DES PAYS-BAS, ELSEVIER SCIENCE PUBLISHERS. AMSTERDAM, NL, VOL. 103, NR. 5, PAGE(S) 145-147 , XP002099866 ISSN: 0165-0513 page 146

## Description

The invention pertains to an immediate-release pharmaceutical dosage form comprising, as the active substance, (7α,17α)-17-hydroxy-7-methyl-19-nor-17-pregn-5(10)-en-20-yn-3-one (tibolone).

Tibolone is known as a tissue-specific and effective agent that can be used in hormone replacement therapy (HRT) in (post)menopausal women, for the treatment of menopausal and postmenopausal disorders, including climacteric complaints, vasomotor symptoms, osteoporosis, and vaginal atrophy. See, int.al., US 5,037,817 and WO 98/47517.

Throughout the past years it has become known that tibolone displays a relatively complex metabolic pattern. Several compounds different from tibolone itself play a role as active metabolites. This makes it difficult to predict whether two different tibolone products are bioequivalent with each other. The bioequivalence of a compound, which exerts its activity through a metabolite, can be measured, in vivo, by analysing plasma levels of the metabolite. However, this is a much more complex matter in the case of a compound such as tibolone, which has more than one metabolite.

In order to set a standard for analysing the relative bioavailability of a drug substance upon oral administration, one method is to choose a model in which, for a dosage amount well within the therapeutic window, a maximum exposure to the drug is given. One viable way to achieve such maximum exposure is to provide the drug in the form of a solution. The normal expectation is that the bioavailability of the drug in solid form will be lower than that in the case of the solution. Sec, int.al., Robert E. Notari, *Biopharmaceutics and Clinical Pharmacokinetics,* 4^{th} edition (1987) p.140.

It has now been found that in the case of tibolone, this experiment led to an unexpected result. For different solid formulations, an analysis was made of two of the main metabolites, viz. 3α-OH tibolone, and 3β-OH tibolone. The finding is that one of these, 3α-OH tibolone, turned out to become better bioavailable as compared to a solution of tibolone, dependent on the particle size of tibolone. As this increase in bioavailability does not hold for the other above-mentioned metabolite, 3β-OH tibolone, obvious explanations could not be given.

Besides being an unexpected result *per* se, this is an improvement of the solid pharmaceutical product as compared to the solution, since 3α-OH tibolone is responsible for an important part of the estrogenic effect of tibolone (which, naturally, plays a role in its significant utility as an HRT agent).

Furthermore this result stresses the difficulty of predicting the bioequivalence of two different dosage forms comprising tibolone.

In EP-A-389035 it was found that the active substance (7α,17α)-17-hydroxy-7-methyl-19-nor-17-pregn-5(10)-en-20-yn-3-one (tibolone) is polymorphous and exists in two crystalline pure forms. Furthermore EP-A-389035 indicates that if the polymorphous compound is used as a medicament, great drawbacks are associated therewith compared with its crystalline pure components. The differences in crystal structure can lead to a difference in physico-chemical parameters such as stability, rate of dissolution, melting point, analytical data and the like, which frequently are strongly influenced by the crystal forms of a polymorphous compound. EP-A-389035 indicates that therefore a crystalline pure form is to be used which purity is greater than 90%.

A pharmaceutical dosage form comprising, as the active substance, (7α,17α)-17-hydroxy-7-methyl-19-nor-17-pregn-5(10)-en-20-yn-3-one (tibolone) is presently available on the European market as 2.5 mg tibolone peroral dosage units under the name Livial®. These dosage units comprise (7α,17α)-17-hydroxy-7-methyl-19-nor-17-pregn-5(10)-en-20-yn-3-one (tibolone) in a crystalline pure form (hereafter referred to as form I) with a purity of at least 98%.

The preparation of a pharmaceutical dosage unit comprising, as the active substance, tibolone, wherein tibolone is crystalline pure, is complex and cumbersome. Not only should the active substance be prepared in a specific crystalline purity but the crystalline purity needs to be maintained throughout an industrial scale production process for pharmaceutical dosage units. Hence, it would be desirable to provide a pharmaceutical dosage unit comprising, as the active substance, polymorphous tibolone. This dosage unit comprising, as the active substance, polymorphous tibolone, should, however, have a similar bioavailability of 3α-OH tibolone as the marketed product.

It has now been surprisingly found that a dosage form comprising polymorphous tibolone, wherein the polymorphous tibolone is present in the dosage form in a mean particle-size of below 22.8 µm has a similar bioavailability of 3α-OH tibolone as the marketed product. This finding enables the use of a polymorphous compound, which can have great economic advantages.

The invention thus resides in pharmaceutical dosage forms, comprising the active substance tibolone as a polymorphous compound, which show an improvement of 3α-OH tibolone availability as compared to a reference situation with maximum exposure, viz. a solution of tibolone, in a similar way as the marketed product.

The immediate-release pharmaceutical dosage forms according to the present invention are characterized by comprising tibolone as a polymorphous compound in a mean particle size of below 22.8 µm in the dosage form as measured by the technique described hereinbelow. Preferably, the mean particle size of the polymorphous tibolone in the dosage form is below 20 µm. Preferably, the pharmaceutical dosage form is a solid, peroral dosage form.

In addition to the above, it was realized that any assessment of bioequivalence of two tibolone products will fail if not 3α-OH tibolone is measured. A method of examining whether one tibolone product is bioequivalent with another tibolone product resides therein that bioequivalency is determined *in vivo* by analysing plasma levels of one or more metabolites of tibolone, one of which being 3α-OH tibolone.

The main requirements for the present invention include that a pharmaceutical composition of polymorphous tibolone is provided, and that this polymorphous tibolone, when contained in the dosage form, has a particle size as defined above.

By polymorphous is understood that the active substance has crystallized in two or more different structures (See e.g. Webster's third new international dictionary, published by Merriam-Webster Inc. 1993). More specific the active substance contains at least two different crystal structures each present in an amount of at least 10% by weight.

For the preparation of tibolone reference is made to US 3,340,279.

Polymorphous tibolone, having the required particle size, can be provided in several ways. A simple, though laborious and not economical method is to just produce polymorphous tibolone in any solid form and screen the obtained particles using adequate mesh sizes so as to sift out the particles that are too large. An alternative method is to first dilute the active substance with a pharmaceutically acceptable carrier (e.g. a base granulate normally used for tabletting or filling of capsules), and then screen the product. In that case the carrier materials effectively work as a processing aid in reducing the size of the active substance (the final maximum particle size of the active substance remains to be determined by the mesh-size of the screen). The necessary equipment as well as these screening methods are known to the skilled man. Another method is to again just produce polymorphous tibolone in any solid form, and then subject it to a milling step wherein the required particle size is obtained. Suitable milling equipment is known to the skilled person. Instead of first producing polymorphous tibolone and then rendering it into the right particle size, one can also take measures during synthesis and, particularly, crystallisation, to actually obtain polymorphous tibolone in the desired particle size. The exact conditions required for this, will depend on the equipment and reaction circumstances used. As a general textbook reference on providing particles of a desired size, referrence is made to Gennaro et al., Remington's Pharmaceutical Sciences, (18th ed., Mack Publishing Company, 1990, pages 1436-1437 and 1615-1632.

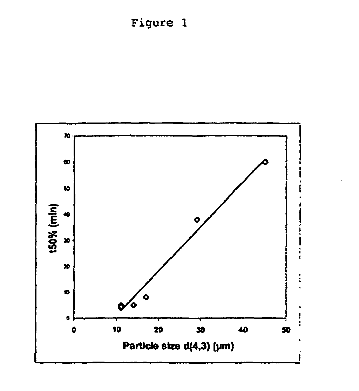

In order to avoid undue experimentation, one can conduct a relatively simple test as to whether the particle size of the polymorphous tibolone, once incorporated into the immediate-release dosage form (be it a compressed tablet, a capsule, or else), is adequate. This is a dissolution test, standardized to a dosage of 2.5 mg tibolone. The dosage form to be tested is put into a dissolution medium (aqueous sodium lauryl sulphate solution 0.25 wt%). By means of a suitable technique, such as HPLC, it is measured at fixed points in time how much tibolone has been dissolved in the medium. This enables determining the dissolution rate of tibolone, given by the t_{50%} value, i.e. the point in time at which 50% of the tibolone, i.e. 1.25 mg, has been dissolved. A t_{50%} value of below 23.1 minutes correlates with a particle size of polymorphous tibolone in compliance with the present invention. It is imperative that the test be standardized to a dosage of 2.5 mg. Figure 1 provides the correlation between the particle size of the polymorphous tibolone and the dissolution rate (t_{50%}) of a solid, immediate-release peroral dosage unit (a tablet) comprising tibolone. Such a correlation is generally valid in the case of immediate-release dosage forms. As indicated above, t_{50%} is the time needed for 50% of the drug to dissolve *in vitro*. The t_{50%} was calculated by means of linear interpolation between the first point >50% of the drug dissolved, and the previous point, sampling points being 5, 10, 15, 30, and 45 minutes after start.

Plain coated tablets, showing a release of the active tibolone which is not deliberately modified by an applied coating, are preferably broken in order to assess the dissolution rate dependant on the particle size of the polymorphous tibolone.

Disintegration is an essential attribute of immediate-release peroral solid dosage units for administration by mouth, except for those intended to be chewed before being swallowed and for some types of extended-release tablets. The disintegrated immediate-release peroral dosage units allow the polymorphous tibolone particles to suspend into the gastro-intestinal contents and to dissolve.

The use of an orally administered liquid immediate-release suspension of solid polymorphous tibolone particles in a mean particle size of below 22.8 µm is also applicable to immediately distribute the solid tibolone particles for dissolution into the gastro-intestinal tract.

If the polymorphous tibolone has been provided in the adequate particle size before mixing with excipients, it can thereafter be mixed with excipients. Eventually, the mixture of polymorphous tibolone and excipients (however obtained) can be incorporated into solid pharmaceutical dosage units in a known manner. The dosage amount of tibolone therein will usually be in the order of 0.3 to 5.0 mg per dosage unit, more particularly 0.625, 1.25 or 2.5 mg.

The pharmaceutical dosage units of the immediate-release type of the present invention will generally take the form of tablets, plain coated tablets or capsules, but other solid or dry pharmaceutical preparations are included. The dosage form of the immediate-release type can also pertain to a liquid suspension of tibolone particles.

Methods for making such dosage units are well known. For example in the above-mentioned Gennaro et al., Remington's Pharmaceutical Sciences, reference is made especially to Part 8: Pharmaceutical Preparations and Their Manufacture), where methods of making tablets, capsules and pills and their respective components are described.

Three methods of making tablets and capsules include the wet-granulation, dry-granulation, and direct compression methods.

Wet-granulation methods involve weighing out ingredients (actives and excipients, including a solvent), mixing the ingredients, granulating them, screening them damp, drying them, dry screening, lubrication, and compressing the resultant admixture into tablets or filling capsules with it. Such procedures result in tablets or capsules having at least adequate homogeneity and stability.

Direct compression methods involve weighing out direct-compression vehicles (including carriers) and active ingredients, mixing of the ingredients, lubrication, and compressing the resulting admixture into tablets, or filling capsules with it.

Carriers for active substances in pharmaceutical dosage units are generally known to the skilled man, and do not require separate elucidation here.

Wet granulation distinguishes from dry granulation and dry-mixing in that a liquid (such as water, preferably containing a binder) is applied in wet granulation to produce agglomeration or granules.

The most widely used granulation methods in the pharmaceutical industry are the fluidised bed granulation and the wet-massing method in which a liquid is added to a powder or granulate in a vessel equipped with any type of agitation that will provide granules or agglomerates. various operations can be recognised in the wet (massing) granulation, including milling of excipients, mixing of milled powders, preparation of binder solution, mixing the binder solution with the powder mixture to form the wet mass, granulation of the mass, coarse screening of wet mass, drying moist granules, and screening dry granules. It is obvious that, depending on the selected excipients and the size of the batch and the selected equipment, some of the operations can be combined or are not required or particular operations can be included. General methods of preparing granules are for instance described in Pharmaceutical Dosage Forms: Tablets (Volume I). Ed. H.A. Lieberman, L. Lachman, J.B. Schwartz (1989), Marcel Dekker Inc. New York and Basel pp. 131-190.

Advantages of wet granulation include improvement of the cohesiveness and compressibility of powders, a good particle size distribution, reduction of a great deal of dust and airborne contamination, prevention of segregation of components.

Small-scale production can be achieved by mixing and wetting the mass in mortars or stainless steel bowls, whereas for larger quantities twin-shell blenders, double-cone blenders, planetary mixers, rotary granulators, high shear mixers and fluid-bed granulation equipment can be applied. General mixing methods are disclosed in Pharmaceutical Dosage Forms (Volume 2). Ed. H.A. Lieberman, L. Lachman, J.B. Schwartz (1990), Marcel Decker Inc. New York and Basel pp. 1-71. The dry excipients and, optionally, active ingredients are mixed in a suitable mixer, preferably a mixer in which both mixing and granulating can be performed, for instance a Gral high shear mixer, after which an aqueous binder solution is added. Another preferred method is suspending the active ingredients into the aqueous binder solution, which suspension is added to the dry mixture of excipients and granulated.

Granulates, tablets, and capsules prepared by wet-granulation or direct compression consist of several inert materials that can be found in conventional solid oral dosage forms in general. The ingredients can be classified in excipients which help to impart satisfactory processing and compression characteristics to the formulation like diluents, stabilising agents, binders, glidants and lubricants and in excipients to give the desirable physical characteristics to the finished tablet like disintegrants and colours. If required the tablets can be provided with a film coat or a sugar coat, for instance as disclosed in Pharmaceutical Dosage Forms (Volume 3). Ed. H.A. Lieberman, L. Lachman, J.B. Schwartz (1990), Marcel Decker Inc. New York and Basel pp. 93-125.

Diluents ("filling excipients") usually make up the major portion of the carrier. Direct compression carriers are described in the same textbook, Volume 1, second edition, Chapter 4, pages 195-246. The direct compression carriers can be classified into groups including water soluble polyalcohols such as lactose (including spray-dried lactose and anhydrous lactose), and polysaccharides such as the group of celluloses (e.g. Avicel® PH 101, PH 102, and PH 200, purified wood cellulose), and the group of water-insoluble starch products according to the invention (e.g. Starch 1500, potato starch, corn starch, wheat starch, including modified starches, agglomerated starches, granulated starches).

Binding agents or adhesives are used as substances that bind powders together and provide cohesiveness to the granulates and tablet formulation. Binders can be added dry and blended with the diluents and, optionally, the drug. In this case binders are activated by addition of water or other solvents. In other manufacturing procedures, the adhesives are dissolved or slurried in a liquid and, in this form, added to the mixed powders. Conventional binders include gelatine, water soluble modified starch, and sugars as sucrose, glucose, dextrose, molasses and lactose. Natural and synthetic gums which have been used include tragacanth, magnesium aluminium silicate, acacia, ammonium calcium alginate, sodium alginate, carboxymethylcellulose, hydroxypropylcellulose, methylcellulose, hydroxypropylmethylcellulose, polyvinylpyrrolidone, polyethylene glycol and clays like Veegum.

Depending on for example the solubility of the binders in the various liquids, the binder can be added to the powder mix as a solution in water, or a water-solvent mixture.

Stabilising agents can be added to reduce decomposition of tibolone if desired. Examples of such stabilising agents are of the group of antioxidants (such as ascorbyl palmitate and ascorbyl stearate) and the group of water soluble chelating agents (such as sodium EDTA and sodium ascorbate).

Materials to improve the flow characteristics are referred to as glidants. As an example, silicon dioxide, magnesium lauryl sulfate, magnesium aluminium silicate, magnesium oxide, talc or clays can be incorporated into the formulation to reduce interparticulate friction and to eliminate the problems associated with the flow of materials from larger to smaller apertures in the tablet presses.

Disintegrants (commonly used in immediate-release dosage forms, and known to the skilled person) can also be used in the dosage forms of the invention.

Before filling capsules or sachets, or compressing tablets, lubricants are mostly added to prevent friction and wear during processing. Some of the lubricants also demonstrate anti-adherent properties that can be relevant in case of sticking of tablet granulations to the faces of the punches and the die walls. Examples of the group of lubricants are the metallic stearates (magnesium stearate), talcum, stearic acid, sodium stearyl fumarate, hydrogenated vegetable oil, and high melting point waxes.

Methods for making suspensions are well established and for instance described in Gennaro et al., Remington's Pharmaceutical Sciences (20^{th} Ed., Lippincott Williams & Wilkins, Baltimore, USA, Chapter 39: Solutions, emulsions, supensions, and extracts).

### Assays

From the above, it is clear that several assays are used in describing the present invention. These are, in arbitrary order, the measurement of mean particle-size, the dissolution test referred to above, the assessment of bioequivalence. These will be described hereinbelow:

### Particle size measurement

### Principle:

The particle size distribution is determined by Laser Diffraction Spectroscopy.

### Equipment:

Laser diffraction particle sizer equipped with a sample dispersion unit.

### Reagents:

1. Polysorbate 80, analytical grade.
2. Water, purified quality (concentration of particles >10 m is < 1 per mL).
3. Polysorbate solution 0.05 %.
   Dissolve 0.5 gram of polysorbate 80 (1) in 1.0 liter of water (2).
4. Suspension liquid.
   Add 50 mg of tibolone sample to 500 ml of polysorbate solution (3) and saturate
   by stirring for at least 60 minutes.
   Filtrate the supernatant liquid and use the filtered solution.

### Instrument conditions:

Particle sizer : Malvern Mastersizer S or equivalent
Lens type : 300RF (range 0.05 µm - 900 µm)
Active beam length : 2.4 mm
Sample dispersion unit : Malvern MS1 or equivalent
Sample flow rate : 3/4 of maximum flow rate Sweeps
   - sample number : 10000
   - background number : 10000
Presentation : Fraunhofer
Analysis model : Polydisperse

### Sample suspension:

Homogenize the sample in the sample container.

Transfer about 30 mg of the sample into a 10 ml glass container.

Add about 2 ml of suspension liquid (4) and close the container.

Suspend the sample in an ultrasonic bath to complete deagglomeration..

### Measurement:

Fill the sample dispersion unit with suspension liquid (4) and measure the blank background.

Add the required amount of the homogeneous sample suspension dropwise into the sample dispersion unit, and analyse.

### Calculation:

Calculate the mean particle size in which the d_{(4,3)} = volume-mean diameter of the particles in µm.

### Dissolution rate of tibolone

### Principle:

After dissolution, tibolone is determined by reversed phase liquid chromatography.

### Equipment:

1. Liquid chromatograph with temperature controlled column compartment and
   variable wavelength absorption detector.
2. Dissolution apparatus 2 according to USP - DISSOLUTION <711> (paddle).

### Reagents and reference standards:

1. Water, HPLC grade USP/ACS suitable for gradient elution analysis or equivalent
   (e.g. milli-Q).
2. Methanol, analytical reagent.
3. Sodium lauryl sulphate solution 99 %, Merck or equivalent.
4. Dissolution medium: Sodium lauryl sulphate solution 0.25 wt %.
   Remove dissolved air from water (1) by purging with helium or equivalent before
   adding sodium lauryl sulphate (3).
   Weigh 10 g of sodium lauryl sulphate (3) and dissolve in 2.0 liter of hot water (1).
   Cool to room temperature and dilute to 4.0 liter with water (1) at room
   temperature.
   Mix carefully, in order to prevent the formation of lather.
5. Tibolone, reference standard.
6. Suitable sinkers in the case of capsules

### Reference solution (in duplicate):

Accurately weigh about 25 mg of tibolone (5) and transfer into a 50 ml volumetric
flask.

Dissolve in and dilute to volume with methanol (2).

Transfer 1.0 ml of this solution into a 100 mL volumetric flask.

Dilute to volume with dissolution medium (4).

Mix carefully, in order to prevent the formation of lather.

### Sample preparation:

Drug substance:

Accurately weigh about 2.5 mg of tibolone and transfer into a gelatine capsule. Add about 20 glass beads, close the capsule and mix gently.

Bring the capsule into the dissolution medium using a pair of tweezers and open the capsule to start dissolution.

For a pharmaceutical dosage form tested, the final amount of tibolone added to the dissolution medium must be 2.5 mg. If the dosage contains less than 2.5 mg tibolone per dosage unit extra dosage units should be added to the medium.

### Tablet:

Add a tablet to the dissolution medium to start dissolution.

### Capsule:

Place the capsule in a suitable sinker and add the sinker with capsule to the dissolution medium to start dissolution.

### Test solution:

Prepare the test solution according to USP - Dissolution <711>.

Withdraw about 10 ml of the test solution using a glass syringe equipped with
stainless steel cannula (or equivalent glass device).

Immediately centrifuge the solution and use the clear supernatant.

### Dissolution conditions:

Apparatus : USP - Dissolution apparatus 2
Dissolution medium : see reagents no. 4
Volume of dissolution medium : 500 ml
Temperature : 37 ± µ0.5 °C
Speed : 0.83 Hz (50 r.p.m.)
Sampling time : 5,10, 15, 30, and 45 minutes after start.

### Chromatographic conditions:

Column : Hypersil ODS, 5 µm, 100 x 4.6 mm or equivalent
Column temperature : 40 °C
Mobile phase : methanol + water (77 + 23 v/v)
Flow rate : 0.5 ml/min
Detector : variable wavelength absorption detector, 205 nm, abs.range about 0.1 AUFS
Injection volume : 200 µl
Number of injections : reference solutions : 3
test solutions : 2
dissolution medium : 1

### System Suitability Test:

Indicating retention time for tibolone is 5.0 minutes.

Alter the water concentration of the mobile phase if any impurity coelutes with
tibolone.

### Criteria:

1. Relative standard deviation of injections of the reference solutions: RSD 3 %.
2. Ratio of the mean response factors of the 2 reference solutions: 0.97 Q 1.03.
3. Response of any peak in the chromatogram of the dissolution medium at the
   position of tibolone: < 3 % with respect to the response of the tibolone peak in the
   chromatogram of the reference solution.

### Calculation:

Calculate the amount of tibolone dissolved in the sample.

### Bioequivalence

### Assay for 3α-OH tibolone.

For the determination of 3α-OH tibolone, deuterated 3α-OH tibolone can be used as internal standard. Plasma samples can be extracted with solid-phase extraction, thereafter silylated and quantified with capillary gas chromatography with mass spectrometric detection in the CI-mode. The lower limit of quantification was 0.1 ng/ml.

### In vivo study

To assess the bioequivalence between a polymorphous tibolone product (indicated as test treatment) and a reference treatment (such as Livial®) a single-dose (2.5 mg) 2-way cross-over study should be performed. The washout period between the two treatments should be at least seven days. The total number of subjects should be at least 22, i.e. 11 subjects per sequence. Subjects should be postmenopausal women, aged 55-65, and physically and mentally healthy. Following single-dose administration, blood samples should be taken at 0 (pre-dose), 0.5, 1, 1.5, 2, 3, 4, 6, 8, 12, 16, 20 and 24 hours post-dosing. The 3α-OH tibolone concentrations should be determined in plasma according to the method described earlier. Based on the 3α-OH tibolone plasma concentrations the peak concentrations (Cₘₐₓ) and the area under the plasma-concentration-versus-time curve from zero to the time point of the last measurable concentration (AUC₀₋ₜₗₐₛₜ) as well as the area under the plasma-concentration-versus-time curve from zero extrapolated to infinity (AUC_{0-∞}) should be calculated. Bioequivalence should be assessed based on Cₘₐₓ, AUC₀₋ₜₗₐₛₜ and AUC_{0-∞} using 90% confidence intervals for the ratio of the test and reference treatment derived from an Analysis of Variance on logₑ-transformed parameter values. The formulations are considered to be bioequivalent if the 90% confidence intervals for the above mentioned ratios of Cₘₐₓ, AUC₀₋ₜₗₐₛₜ and AUC_{0-∞} are fully contained within the acceptance range 0.80 - 1.25.

The invention will be illustrated hereinafter with reference to the following Examples and the Figures.

### Example 1:

Solid dosage forms of polymorphous tibolone are made by a process similar to that described in US 5,037,817.

| Composition of the tablets: | |
|---|---|
| Ingredient | Quantity |
| Tibolone | 2.5 mg |
| Potato starch | 10 mg |
| Ascorbyl palmitate | 0.2 mg |
| Magnesium stearate | 0.5 mg |
| Lactose | 86.8 mg |
| Tablet mass | 100 mg |

A basic granulate is prepared by granulation of a mixture of lactose (diluent), potato starch (90% of the total amount; disintegrant) and potato starch mucilage (10% of the total amount; binder) in a fluid bed granulator. After granulation, the basic granulate is passed through a sieve. Part of the granulate (approx. 10% w/w of the final batch size) is mixed with tibolone and ascorbyl palmitate using a suitable blender and then passed through a premix sieve. The tibolone premix and the remainder of the basic granulate are mixed in a suitable blender. Magnesium stearate is added and mixed. The mixture is compressed into 100 mg tablets.

Thus three batches (batch A, B and C) have been prepared, the only material difference between them being analysed as the particle size of the polymorphous tibolone used. See the table below.

The table furthermore includes a batch (batch M) of solid dosage forms comprising tibolone, which is representative for the marketed product

### Example 2:

A solution of tibolone is made as follows. Tibolone is dissolved into Ethanol 96%. The final concentration of tibolone in solution is 1 mg/ml. The solution is mixed and filled in 5 ml vials; 2,5 ml/vial. Dilute instruction: The content of the vial must be diluted with 2.5 ml of solvent (water for injection) up to a total volume of 5 ml before use.

| Table of Tibolone Drug Product Batches | | | | | |
|---|---|---|---|---|---|
| Batch | Initial particle size tibolone d_{(4,3)} (µm) | Premix sieve (µm) | Mean particle size of tibolone in dosage form (µm) | form I (%) | form II (%) |
| M | - | - | 14 µm | 98 | 2 |
| A | 45 | 125 | 19.3 µm | 89 | 11 |
| B | 102 | 125 | 22.8 µm | 79 | 21 |
| C (comparative) | 102 | 250 | 48.2 µm | 79 | 21 |
| S (solution) | Solution according to Example 2 | | | | |

### Example 3

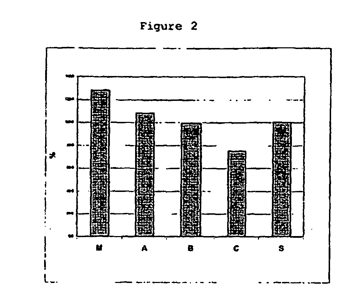

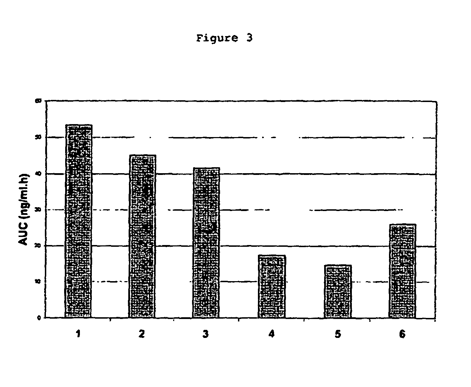

The tibolone batches of Examples 1 and 2 are tested for the relative bioavailability of 3α-OH tibolone. This test is conducted in the form of the single-dose (2.5 mg) 2-way cross-over study as described hereinbefore, exactly complying with the requirements thereof. The results are depicted in Figures 2 and 3.

Figure 1 provides the correlation between the particle size of polymorphous tibolone and tablet-dissolution described hereinbefore of tablets prepared according to Example 1.

In Figure 2 the relative bioavailability of 3α-OH tibolone is shown. On the Y-axis is depicted the relative Area Under the plasma-concentration versus time Curve (AUC), with the solution (batch S) being the reference at 100%. From the picture it is clear that batches M, A and B show an unexpected bioavailability of 3α-OH tibolone at least equal to that of the solution batch S.

In Figure 3 the AUC of 3α-OH tibolone and 3β-OH tibolone is shown for batches M, A and S, as follows:
(1) is a bar indicating the AUC for 3α-OH tibolone after the administration of batch M;
(2) is a bar indicating the AUC for 3α-OH tibolone after the administration of batch A;
(3) is a bar indicating the AUC for 3α-OH tibolone after the administration of batch S;
(4) is a bar indicating the AUC for 3β-OH tibolone after the administration of batch M;
(5) is a bar indicating the AUC for 3β-OH tibolone after the administration of batch A;
(6) is a bar indicating the AUC for 3β-OH tibolone after the administration of batch S;

From the relative height of the bars, one can see that the effect of enhanced bioavailability of 3α-OH tibolone resulting from the administration of batches M and A, is not observed for 3β-OH tibolone.

## Claims

1. An immediate-release pharmaceutical dosage form comprising, as the active substance, polymorphous tibolone and pharmaceutically acceptable excipients, wherein the polymorphous tibolone has a mean particle size of below 22.8 µm in the dosage form.

2. A pharmaceutical dosage form according to claim 1, wherein the mean particle size of the polymorphous tibolone in the dosage form is below 20 µm.

3. A method of producing a pharmaceutical dosage form according to claim 1 or 2 comprising:
obtaining tibolone particles of polymorphous tibolone having a mean particle size of below 22.8 µm in the dosage form;
mixing said tibolone particles with at least one pharmaceutically acceptable excipient to obtain an admixture; and
compressing said admixture to form a solid pharmaceutical formulation.

4. Method according to claim 3 wherein obtaining said tibolone particles comprises milling solid polymorphous tibolone to produce tibolone particles having a mean particle size below 22.8 µm in the dosage form.

5. Method according to claim 3, wherein obtaining said tibolone particles comprises synthesizing polymorphous tibolone particles having a mean particle size below 22.8 µm in the dosage form.

6. Method according to anyone of claims 3 to 5, wherein obtaining said tibolone particles comprises obtaining tibolone particles having a mean particle size below 20 µm in the dosage form.

7. Use of polymorphous tibolone for the manufacture of a medicament for the treatment or prevention of menopausal and post-menopausal complaints including osteoporosis, wherein the polymorphous tibolone has a mean particle size as defined in the specification of below 22.8 µm in the dosage form.

## Patentansprüche

1. Schnell freisetzende pharmazeutische Darreichungsform mit polymorphem Tibolon als Wirkstoff und mit pharmazeutisch verträglichen Hilfsstoffen, wobei das polymorphe Tibolon in der Darreichungsform eine mittlere Partikelgrösse von unter 22,8 Mikrometer aufweist.

2. Pharmazeutische Darreichungsform nach Anspruch 1, bei der die mittlere Partikelgrösse des polymorphen Tibolon in der Darreichungsform unter 20 Mikrometer ist.

3. Verfahren zur Herstellung einer pharmazeutischen Darreichungsform nach Anspruch 1 oder Anspruch 2, mit den Schritten:
- des Erhaltens von Tibolonpartikeln aus polymorphem Tibolon mit einer mittleren Partikelgrösse von unter 22,8 Mikrometer in der Darreichungsform,
- des Mischens der besagten Tibolonpartikel mit mindestens einem pharmazeutisch verträglichen Hilfsstoff, um eine Beimischung zu erhalten, und
- des Komprimierens der besagten Beimischung, um eine feste pharmazeutische Formulierung zu erhalten.

4. Verfahren nach Anspruch 3, bei dem das Erhalten der besagten Tibolonpartikel das Mahlen von festem polymorphen Tibolon umfasst, um Tibolonpartikel mit einer mittleren Partikelgrösse von unter 22,8 Mikrometer in der Darreichungsform zu erzeugen.

5. Verfahren nach Anspruch 3, bei dem das Erhalten der besagten Tibolonpartikel das Synthetisieren von polymorphen Tibolonpartikeln umfasst, die eine mittlere Partikelgrösse von unter 22,8 Mikrometer in der Darreichungsform aufweisen.

6. Verfahren nach einem der Ansprüche 3 bis 5, bei dem das Erhalten der besagten Tibolonpartikel das Erhalten von Tibolonpartikeln mit einer mittleren Partikelgrösse von unter 20 Mikrometer in der Darreichungsform umfasst.

7. Verwendung von polymorphem Tibolon zur Herstellung eines Medikamentes für die Behandlung oder die Vermeidung von menopausalen und post-menopausalen Beschwerden umfassend Osteoporose, wobei das polymorphe Tibolon in der Darreichungsform eine mittlere Partikelgrösse wie in der Beschreibung definiert von unter 22,8 Mikrometer aufweist.

## Revendications

1. Une forme de dosage galénique à libération immédiate comprenant, en tant que substance active, de la tibolone polymorphe et des excipients pharmaceutiquement acceptables, la tibolone polymorphe présentant, dans ladite forme de dosage, une dimension particulaire moyenne inférieure à 22,8 µm.

2. Une forme de dosage galénique selon la revendication 1, dans laquelle la dimension particulaire moyenne de la tibolone polymorphe, dans ladite forme de dosage, est inférieure à 20 µm.

3. Une méthode de production d'une forme de dosage galénique selon la revendication 1 ou 2, comprenant :
- l'obtention, dans ladite forme de dosage, de particules de tibolone polymorphe présentant une dimension particulaire moyenne inférieure à 22,8 µm ;
- le mélange desdites particules de tibolone avec au moins un excipient pharmaceutiquement acceptable pour obtenir un mélange ; et
- la compression dudit mélange pour former une formulation pharmaceutique solide.

4. La méthode selon la revendication 3, dans laquelle l'obtention desdites particules de tibolone comprend le broyage de tibolone polymorphe solide pour produire, dans la forme de dosage, des particules de tibolone présentant une dimension particulaire moyenne inférieure à 22,8 µm.

5. La méthode selon la revendication 3, dans laquelle l'obtention desdites particules de tibolone comprend la synthèse de particules de tibolone polymorphe présentant, dans la forme de dosage, une dimension particulaire moyenne inférieure à 22,8 µm.

6. La méthode selon l'une quelconque des revendications 3 à 5, dans laquelle l'obtention desdites particules de tibolone comprend, dans la forme de dosage, l'obtention de particules de tibolone présentant une dimension particulaire moyenne inférieure à 20 µm.

7. Emploi de tibolone polymorphe pour la fabrication de médicaments pour le traitement ou la prévention des troubles de la ménopause et de la post-ménopause, y compris l'ostéoporose, dans lequel la tibolone polymorphe présente une dimension particulaire moyenne, dans la forme de dosage telle que définie dans la présente description, inférieure à 22,8 µm.
